# EUROPÄISCHE PATENTSCHRIFT

(11) **EP 2 723 749 B1**
(45) Veröffentlichungstag und Bekanntmachung des Hinweises auf die Patenterteilung: **18.03.2015**
(21) Anmeldenummer: 12719329.0
(22) Anmeldetag: 26.04.2012
(51) Int. Cl.: C07D 493/04, C08K 5/1535

(54) **DIANHYDROHEXITOLDIESTER DER 2-ETHYLHEPTANSÄURE**
DIANHYDROHEXITOL DIESTERS OF 2-ETHYLHEPTANOIC ACID
DIESTERS DE DIANHYDROHEXITOL D'ACIDE 2-ÉTHYLHEPTANE

(30) Priorität: 21.06.2011 DE 102011077857
(43) Veröffentlichungstag der Anmeldung: 30.04.2014
(73) Patentinhaber: Evonik Degussa GmbH, 45128 Essen (DE)
(72) Erfinder: GEVERS, Andreas, 46240 Bottrop (DE); BECKER, Hinnerk, Gordon, 45257 Essen (DE); GRASS, Michael, 45721 Haltern am See (DE); WINTERBERG, Markus, 45711 Datteln (DE); MASCHMEYER, Dietrich, 45657 Recklinghausen (DE); BÖING, Christian, 50679 Köln (DE)
(86) Internationale Anmeldenummer: PCT/EP2012/057639
(87) Internationale Veröffentlichungsnummer: WO 2012/175237

(56) Entgegenhaltungen:
- WO-A1-01/83488
- WO-A1-99/45060
- WO-A1-2008/155159

## Beschreibung

Die vorliegende Erfindung betrifft Dianhydrohexitoldiester der 2-Ethylheptansäure. Ebenfalls betrifft die vorliegende Erfindung ein Gemisch, welches einen solchen Ester umfasst, sowie die Verwendung des Esters und des Gemisches.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Diisononylphthalat (DINP) und Diisodecylphthalat (DIDP) Verwendung finden. Daneben werden beispielsweise auch alicyclische Polycarbonsäureester, wie die Ester der Cyclohexan-1,2-dicarbonsäure als Weichmacher für Kunststoffe wie Polyvinylchlorid (PVC), Polyvinylbutyral (PVB) und Polyolefine eingesetzt, da sie in Bezug auf die Gesundheitsgefährdung als unbedenklicher eingeschätzt werden, als die entsprechenden Phthalsäureester.

In der EP 2 114 952 A0 wird ein Gemisch von Dianhydrohexitoldiestern beschrieben, in dem zumindest zwei unterschiedliche Diester enthalten sind, welche sich in der Konstitution zumindest eines der vorhandenen Carbonsäurereste unterscheiden.

Weiter beschreiben die Dokumente WO 01/83488, WO 99/45060 und WO 2008/155159 Dianhydrohexitoldiester der 2-Ethylhexansäure, deren Gemische und deren Verwendung als Weichmacher.

Ausgehend von dem bekannten Stand der Technik bestand die Aufgabe der vorliegenden Erfindung in der Bereitstellung einer Verbindung, mit einem genau definierten Säureanteil, welcher nicht variiert. Hierbei sollte die Verbindung im Hinblick auf ausgewählte physikalische Parameter bessere, oder mindestens genau so gute Weichmachereigenschaften aufweisen, als das in EP 2 114 952 A0 beschriebene Gemisch.

Estergemische haben oft den Nachteil, dass diese nicht genau definiert sind, oder sich in ihrer Zusammensetzung von einer Charge zur nächsten ändern können.

Das Problem wird gelöst durch eine Verbindung gemäß der Formel I: 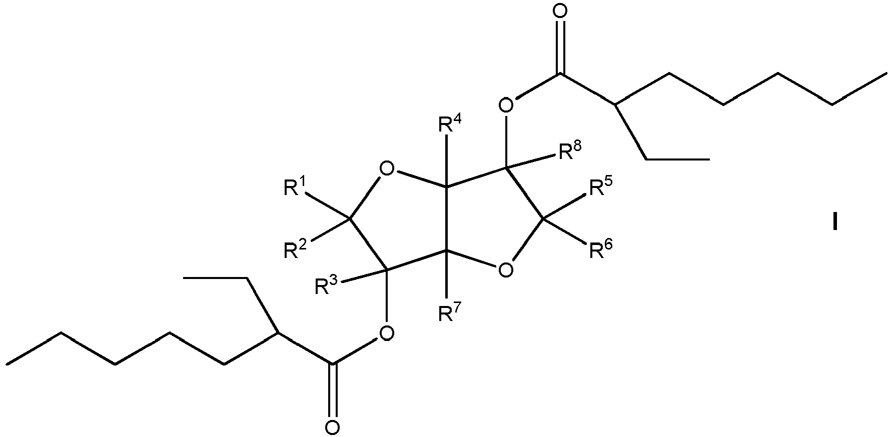 mit R¹ bis R⁸ = H oder Alkylgruppe mit 1 bis 6 Kohlenstoffatomen, wobei die Reste R¹ bis R⁸ gleich oder unterschiedlich sein können.

In einer Ausführungsform der Erfindung entsprechen sich die Reste R¹ und R⁶, sowie R² und R⁵ jeweils.

In einer Ausführungsform der Erfindung sind die Reste R¹, R², R⁵, R⁶ jeweils H.

In einer Ausführungsform der Erfindung entsprechen sich die Reste R⁴ und R⁷.

In einer Ausführungsform der Erfindung sind die Reste R⁴, R⁷ jeweils H.

In einer Ausführungsform der Erfindung sind die Reste R¹ bis R⁸ jeweils H.

In einer Ausführungsform der Erfindung ist mindestens einer der Reste R¹ bis R⁸ nicht H.

In einer Ausführungsform der Erfindung weist die Verbindung ein Inversionszentrum auf.

In einer Ausführungsform der Erfindung weist die Verbindung kein Inversionszentrum auf.

Neben der Verbindung selbst wird auch ein Gemisch beansprucht, welches zumindest eine der zuvor beschriebenen Verbindungen umfasst.

In einer Ausführungsform umfasst das Gemisch:
- eine Verbindung, wie sie zuvor beschrieben wurde,
- ein Polymer.

Das Polymer kann beispielsweise ausgewählt sein aus: Polyvinylchlorid (PVC), Polyvinylbutyral (PVB) und die Polyalkylmethacrylate (PAMA).

In einer weiteren Ausführungsform beträgt das Massen-Verhältnis von Polymer zur Verbindung der Formel I von 30 zu 1 bis 1 zu 2,5.

In einer weiteren Ausführungsform umfassend das Gemisch zusätzlich:
- einen Weichmacher, welcher nicht der Formel I entspricht.

Der Weichmacher, welcher nicht der Formel I entspricht, kann beispielsweise ausgewählt sein aus den Citronensäuretrialkylestern, acylierten Citronensäuretrialkylestern, Glycerinestern, Glycoldibenzoaten, Alkylbenzoaten, Dialkyladipaten, Trialkyltrimellitaten, Dialkylterephthalaten, Dialkylphthalaten oder den Dialkylestern der 1,2-, 1,3- oder 1,4-Cyclohexandicarbonsäuren, wobei die Alkylreste von 4 bis 13, vorzugsweise 5, 6, 7, 8, 9, 10, 11, 12 oder 13 Kohlenstoffatome aufweisen. Die Weichmacher können auch Dianhydrohexitolester, bevorzugt Isosorbiddiester, anderer Carbonsäuren, wie zum Beispiel n- oder iso-Buttersäure, Valeriansäure oder 2-Ethylhexansäure sein.

In einer weiteren Ausführungsform beträgt das Mol-Verhältnis von dem Weichmacher, welcher nicht der Formel I entspricht, zu der Verbindung gemäß der Formel I von 1 zu 10 bis 10 zu 1.

In bevorzugten Gemischen, die die Verbindung gemäß der Formel I und Weichmacher, die nicht der Formel I entsprechen, aufweisen, beträgt das Mol-Verhältnis von Weichmachern, zu der Verbindung gemäß Formel I vorzugsweise von 1 zu 10 bis 10 zu 1, bevorzugt von 1 zu 6 bis 6 zu 1. Bei den Weichmachern handelt es sich vorzugsweise um einen Weichmacher ausgewählt aus: Alkylbenzoaten, Dialkyladipaten, Citronensäuretrialkylestern, acylierten Citronensäuretrialkylestern, Trialkyltrimellitaten, Glycoldibenzoaten, Dialkylterephthalaten, Dialkylphthalaten, Dialkanoylestern des Isosorbid, Dialkylestern der 1,2-, 1,3- oder 1,4-Cyclohexandicarbonsäuren.

In einer weiteren Ausführungsform ist das Polymer Polyvinylchlorid (PVC).

In einer weiteren Ausführungsform weist das Gemisch keine unterschiedlichen Diester auf, die sich in der Konstitution zumindest eines der vorhandenen Carbonsäurereste C₈H₁₇COO unterscheiden, auf.

Neben dem Gemisch als solches wird auch dessen Verwendung als Weichmacher beansprucht.

In einer weiteren Ausführungsform werden die erfindungsgemäßen Gemische als Weichmacher in Plastisolen, Klebstoffen oder Klebstoffkomponenten, in Dichtungsmassen verwendet. Bevorzugte Plastisole sind dabei insbesondere PVC-oder PAMA-Plastisole.

Bevorzugte Kunststoffe sind dabei insbesondere Polyvinylchlorid (PVC), Polyvinylbutyral (PVB), Homo- und Copolymere auf Basis von Ethylen, Propylen, Butadien, Vinylacetat, Celluloseacetat, Glycidylacrylat, Glycidylmethacrylat, Methacrylaten, Acrylaten, Acrylaten mit am Sauerstoffatom der Estergruppe gebundenen Alkylresten von verzweigten oder unverzweigten Alkoholen mit einem bis zehn Kohlenstoffatomen, Styrol, Acrylnitril, Homo- oder Copolymere von cyclischen Olefinen.

In einer weiteren Ausführungsform werden die erfindungsgemäßen Gemische als Lösungsmittel in Kunststoffen oder Kunststoffkomponenten verwendet.

Der Kunststoff kann hierbei beispielsweise ausgewählt sein aus: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, Polymethacrylat, Polymethylmethacrylat, Methylacrylat-Butylacrylat-Copolymere, Methylmethacrylat-Butylmethacrylat-Copolymere oder allgemein Polyalkylmethacrylate (PAMA), Ethylen-Vinylacetat-Copolymere, chloriertes Polyethylen, Nitrilkautschuk, AcrylnitrilButadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere, Celluloseacetat, PVB und PVC. Ein besonders bevorzugter Kunststoff ist dabei PVC.

In einer weiteren Ausführungsform wird das erfindungsgemäße Gemisch in Farben, Tinten oder Lacken verwendet.

In einer weiteren Ausführungsform wird das erfindungsgemäße Gemisch als Lösemittel, als Schmierölkomponente, als Kühlflüssigkeit, Bohrflüssigkeit oder Bestandteil davon oder als Hilfsmittel bei der Metallverarbeitung verwendet.

Darüber hinaus können die erfindungsgemäßen Gemische zur Modifizierung von Kunststoffmischungen, beispielsweise der Mischung eines Polyolefins mit einem Polyamid, eingesetzt werden.

Zusammensetzungen aus Kunststoff/-en, insbesondere PVC oder PAMA, die erfindungsgemäße Gemische umfassen sind beispielsweise in folgenden Produkten: Gehäuse für Elektrogeräte, wie beispielsweise Küchengeräte, Computergehäuse, Gehäuse und Bauteile von Phono- und Fernsehgeräte, Rohrleitungen, Apparate, Kabel, Drahtummantelungen, Isolierbänder, im Innenausbau, im Fahrzeug- und Möbelbau, Plastisolen, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, Beflockungen und Bedruckungen, Fasern für Gewebe, beschichtete Gewebe. Weiterhin können Zusammensetzungen aus Kunststoff, insbesondere PVC, die erfindungsgemäße Gemische, die Ester der Formel I enthalten oder aus ihnen bestehen, beispielsweise zur Herstellung folgender Erzeugnisse verwendet werden: Rohrleitungen, Schläuche, Kabel, Draht-Ummantelungen, Isolierbänder, im Fahrzeug- und Möbelbau, Plastisole, Profile, Bodenbeläge, medizinische Artikel (wie z. B. Blutbeutel, Schläuche, Infusionsbeutel etc), Spielzeuge, Lebensmittelverpackungen, Dichtungen, Folien, Verbundfolien, Platten, Kunstleder, Tapeten, Verpackungsbehälter, Klebebandfolien, Bekleidung, Beschichtungen oder Fasern für Gewebe, Schuhe, Unterbodenschutz, Nahtabdichtungen, Dachbahnen, Modelliermassen oder Bälle.

PVC-Zusammensetzungen oder Plastisole, die PVC und ein erfindungsgemäßes Gemisch aufweisen, enthalten vorzugsweise von 5 bis 250 Massenteile, bevorzugt von 10 bis 200 Massenteile und besonders bevorzugt von 20 bis 100 Massenteile an dem erfindungsgemäßen Gemisch pro 100 Massenteilen PVC.

Die erfindungsgemäße Verbindung kann beispielsweise durch die Veresterung von 2-Ethylheptansäure mit Isosorbid erhalten werden.

### Beispiel 1: Herstellung von Isosorbid-di-2-ethylheptanoat (erfindungsgemäß)

In einem 2 Liter-Mehrhalskolben mit Rührer, Wasserabscheider, Tropftrichter, Innenthermometer und Tauchrohr wurden 365 g (2,5 Mol) Isosorbid (Fa. Roquette) mit 948 g (6,0 Mol) 2-Ethylheptansäure und 2,19 g H₃PO₂ (0,6 Massen-% bezogen auf Isosorbid) unter Rühren und Stickstoffeinperlung über das Tauchrohr (6 l/h) bis zum Sieden erhitzt. Im Verlauf der Reaktion stieg die Temperatur auf 240 °C. Ab 240 °C wurde durch Zugabe von Cyclohexan die Temperatur konstant gehalten und gewährleistet, dass das Reaktionswasser über den Wasserabscheider komplett ausgetragen werden konnte. Der Reaktionsverlauf wurde über GC-Analytik verfolgt. Nach insgesamt etwa 14,5 Stunden war die Reaktion beendet und der Wasserabscheider wurde dann durch eine Destillationsbrücke ersetzt, über die bei einer Temperatur bis 200 °C und einem Vakuum von < 1 mbar die überschüssige 2-Ethylheptansäure abdestilliert werden konnte. Danach wurde das Vakuum über Einperlung von Stickstoff (bei 200 °C) auf 40 mbar eingestellt. Bei konstanter Temperatur wurde das Produkt zwei Stunden im Stickstoffstrom gereinigt. Danach wurde der Ansatz, unter einperlen von Stickstoff, auf 80 °C abgekühlt und über eine Nutsche mit Filterpapier und Filterhilfsmittel (Filterperlite Typ: D14) gefiltert. Anschließend wurde das Produkt bei 80 °C mit 2 % basischem Aluminiumoxid (Sigma-Aldrich) bezogen auf die Estermenge neutralisiert. Die so erhaltene Verbindung hatte eine Farbzahl nach Hazen von APHA 12, die Säurezahl war 0,056 mg KOH/g. Die Reinheit, bestimmt über GC-Analytik, lag bei 98,9%.

Das in den nachfolgenden Beispielen als Vergleichsprodukt herangezogene nicht erfindungsgemäße Isosorbid-diisononanoat wurde analog zu Beispiel 1, jedoch aus Isosorbid und Isononansäure hergestellt.

### Beispiel 2: Herstellung von Isosorbid-di-isononanoat (Vergleichsbeispiel)

In einem 4 Liter-Mehrhalskolben mit Rührer, Wasserabscheider, Tropftrichter, Innenthermometer und Tauchrohr wurden 803 g (5,5 Mol) Isosorbid (Fa. Roquette) mit 2212 g (14 Mol) frisch destillierter Isononansäure (hergestellt gemäß WO2008/095571 Beispiel 1) und 4,82 g H₃PO₂ (0,6 Massen-% bezogen auf Isosorbid) unter Rühren und Stickstoffeinperlung über das Tauchrohr (6 l/h) bis zum Sieden erhitzt. Im Verlauf der Reaktion stieg die Temperatur auf 240 °C. Ab 240 °C wurde durch erhöhen des Stickstoffstroms die Temperatur konstant gehalten und gewährleistet, dass das Reaktionswasser über den Wasserabscheider komplett ausgetragen werden konnte. Der Reaktionsverlauf wurde über GC-Analytik verfolgt. Nach insgesamt etwa 9 Stunden war die Reaktion beendet. Dem Reaktionsaustrag wurde 1 % Aktivkohle (CAP Super von Norit) bezogen auf die Einwaage zugesetzt und der Wasserabscheider wurde durch eine Destillationsbrücke ersetzt, über die bei einer Temperatur bis 215 °C und einem Vakuum von < 1 mbar die überschüssige Isononansäure abdestilliert wurde. Danach wurde das Vakuum über Einperlung von Stickstoff (bei 200 °C) auf 40 mbar eingestellt. Bei konstanter Temperatur wurde das Produkt zwei Stunden im Stickstoffstrom gereinigt. Danach wurde der Ansatz, unter einperlen von Stickstoff, auf 80 °C abgekühlt und über eine Nutsche mit Filterpapier und Filterhilfsmittel (Filterperlite Typ: D14) gefiltert. Anschließend wurde das Produkt bei 80 °C mit 2 % basischem Aluminiumoxid (Sigma-Aldrich) bezogen auf die Estermenge neutralisiert. Die so erhaltene Verbindung hatte eine Farbzahl nach Hazen von APHA 13, die Säurezahl war 0,054 mg KOH/g. Die Reinheit, bestimmt über GC-Analytik, lag bei 99,8 %.

Das erfindungsgemäße Produkt und das Vergleichsprodukt wurden im Anschluss in PVC-Formulierungen auf ihre Eignung als Weichmacher überprüft.

### Beispiel zur Herstellung von Plastisolen

Die verwendeten Einwaagen der Komponenten für die verschiedenen Plastisole sind der nachfolgenden Tabelle 1 zu entnehmen.

**Tabelle 1: Plastisolrezeptur (alle Angaben in phr (= Massenteile pro 100 Massenteile PVC))**

| **Rezeptur:** | Formulierung 1 (erfindungsgemäß) | Formulierung 2 (Vergleichsverbindung) |
|---|---|---|
| B 7021 Ultra | 100 | 100 |
| Isosorbiddiisononanoat | 50 | |
| Isosorbid-di-2-ethylheptanoat | | 50 |
| Drapex 39 (epoxidiertes Sojabohnenöl, Fa. Galata) | 3 | 3 |
| Mark CZ 149 (Ca-Zn-Stabilisator, Fa. Galata | 2 | 2 |

Hierbei gilt für beide Ester R¹ bis R⁸ gleich H.

Die Weichmacher wurden vor der Zugabe auf 25 °C temperiert. Zuerst wurden die flüssigen Bestandteile und dann die pulverförmigen in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

### Messung der Plastisol-Viskositäten

Die Messung der Viskositäten der wie zuvor beschrieben hergestellten Plastisole wurden in Anlehnung an die DIN 53 019 mit einem Rheometer Physica DSR 4000 (Fa. Paar-Physica), welches über die zugehörige Software US 200 gesteuert wird, wie folgt durchgeführt: Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die oben genannte Software. Folgende Punkte wurden angesteuert:
- Eine Vorscherung von 100 s⁻¹ für den Zeitraum von 60 s, bei der keine Messwerte aufgenommen wurden
- Eine Abwärtsrampe, beginnend bei 200 s⁻¹ bis herunter zu 0,1 s⁻¹, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Zwischen den Messungen wurde die Paste bei 25 °C gelagert.

In der nachfolgenden Tabelle 2 sind jeweils die Viskosität für die PVC-Paste auf Basis des erfindungsgemäßen Isosorbid-di-2-ethylheptansäureester (1) und als Vergleichsbeispiel auf Basis eines nicht erfindungsgemäßen Isosorbid-di-isononanoylester (2) in Abhängigkeit von der Schergeschwindigkeit in 100 s⁻¹ aufgeführt.

**Tabelle 2: Viskositäten von (1) und (2) jeweils in Pa*s:**

| Schergeschwindgkeit [100 s⁻¹] | **(1)** | **(2)** |
|---|---|---|
| 8,61 | 5,17 | 5,24 |
| 6,63 | 4,58 | 4,7 |
| 5,1 | 4,11 | 4,27 |
| 3,92 | 3,75 | 3,95 |
| 3,02 | 3,48 | 3,71 |
| 2,32 | 3,28 | 3,54 |
| 1,79 | 3,14 | 3,43 |
| 1,37 | 3,05 | 3,36 |
| 1,06 | 2,99 | 3,32 |
| 0,814 | 2,97 | 3,31 |
| 0,626 | 2,95 | 3,34 |
| 0,482 | 2,98 | 3,37 |
| 0,371 | 3,01 | 3,44 |
| 0,285 | 3,06 | 3,54 |
| 0,22 | 3,14 | 3,65 |
| 0,169 | 3,24 | 3,77 |
| 0,13 | 3,37 | 3,91 |
| 0,1 | 3,5 | 4,07 |

Überraschender weise konnte festgestellt werden, dass die PVC-Pasten auf Basis des erfindungsgemäßen Isosorbidesters der 2-Ethylheptansäure (1) im Vergleich zu der analogen Paste auf Basis des nicht erfindungsgemäßen Isosorbidesters der Isononansäure (2) (entsprechend EP 2 114 952 A0) über den gesamten getesteten Scherbereich eine geringere Viskosität und hierdurch eine verbesserte Verarbeitbarkeit aufweist. Hiermit weisen die erfindungsgemäßen Ester bessere Weichmachereigenschaften auf, als der Vergleichsester, welcher einem Ester entspricht, wie er in der EP 2 114 952 A0 beschrieben wurde.

### Messung der Thermostabilität

Die Thermostabilitätsmessungen wurden an einem Mathis Thermotester (Typ LTE-TS Fa. Mathis AG) durchgeführt. Der Probenrahmen für die Thermostabilitätsmessung ist mit 14 Aluschienen bestückt. Die Aluschienen dienen als Probenhalter, in denen Proben bis zu einer Maximalbreite von 2 cm gelegt werden.

Die Ränder der zu untersuchenden Folien wurden mit Hilfe einer Schlagschere entfernt und die Folien wurden rechtwinklig geschnitten (Maße: 20 cm x 30 cm). Dann wurden zwei Streifen (20 * 2 cm) abgeschnitten. Die Streifen wurden nebeneinander in die Aluschienen des Rahmens für die Thermostabilitätsmessung gelegt, und mit der Metallklammer befestigt. Die 3 äußersten Schienen im Rahmen wurden nicht belegt, da die Temperatur in der Mitte des Mathis Ofens am gleichmäßigsten ist.

Am Mathis Thermotester wurden folgende Parameter eingestellt:
Temperatur: 200 °C
Intervallvorschub: 28 mm
Intervallzeit: 1 min
Lüfterumdrehungszahl: 1800 U/min

Nach eingeregelter Temperatur wurde der Rahmen in der Führung des Thermotesters eingerastet und die Messung gestartet. Mit Hilfe eines Byk Farbmessgerätes (Spectro Guide 45/0 Fa. Byk Gardner) wurden bei den abgekühlten Teststreifen die L* a* b* Werte, incl. des Gelbwertes Yi nach Index D1925 ermittelt. Die eingestellte Lichtart C/2° und die Verwendung eines Probenbeobachters wurden benutzt um optimale Messergebnisse zu erzielen. Die Thermostabilitätsstreifen wurden nun bei jedem Vorschub (28 mm) vermessen. Da die Thermostabilitätsstreifen aus zwei 20 cm Streifen bestehen, wurde der Messwert an der Schnittstelle nicht bestimmt. Die Messwerte wurden direkt auf der Musterkarte hinter einer weißen Kachel bestimmt.

In der nachfolgenden Tabelle 3 sind jeweils die Gelbwerte Yi der Thermostabilitäten für eine PVC-Pasten auf Basis des erfindungsgemäßen Isosorbid-di-2-ethylheptansäureester (1) und als Vergleichsbeispiel auf Basis eines nicht erfindungsgemäßen Isosorbid-di-isononansäureester (2) in Abhängigkeit von der Verweilzeit in Minuten aufgeführt.

**Tabelle 3: Gelbwert Yi von (1) und (2)**

| Verweilzeit [min] | **(1)** | **(2)** |
|---|---|---|
| 1 | 1,90 | 2,08 |
| 2 | 2,92 | 2,23 |
| 3 | 3,41 | 3,29 |
| 4 | 4,11 | 4,60 |
| 5 | 4,54 | 7,05 |
| 6 | 5,07 | 12,06 |
| 8 | 9,85 | 36,24 |
| 9 | 18,05 | 54,56 |
| 10 | 33,72 | 72,73 |
| 11 | 57,88 | 90,82 |
| 12 | 85,39 | 139,49 |

Überraschender weise konnte festgestellt werden, dass die PVC-Pasten auf Basis des erfindungsgemäßen Isosorbidesters der 2-Ethylheptansäure (**1**) im Vergleich zu der analogen Paste auf Basis des nicht erfindungsgemäßen Isosorbidesters der Isononansäure (**2**) (entsprechend EP 2 114 952 A0) eine bessere Thermostabilität aufweist. Hiermit weisen die erfindungsgemäßen Ester bessere Weichmachereigenschaften auf, als der Vergleichsester, welcher einem Ester entspricht, wie er in der EP 2 114 952 A0 beschrieben wurde.

Anhand der Messwerte für die Viskosität und für den Gelbwert konnte aufgezeigt werden, dass die erfindungsgemäße Verbindung beziehungsweise ein Gemisch, welches eine solche Verbindung umfasst, bessere Weichmachereigenschaften im Hinblick auf ausgewählte physikalische Parameter aufweist als die Vergleichsverbindung.

## Patentansprüche

1. Verbindung gemäß der Formel I: 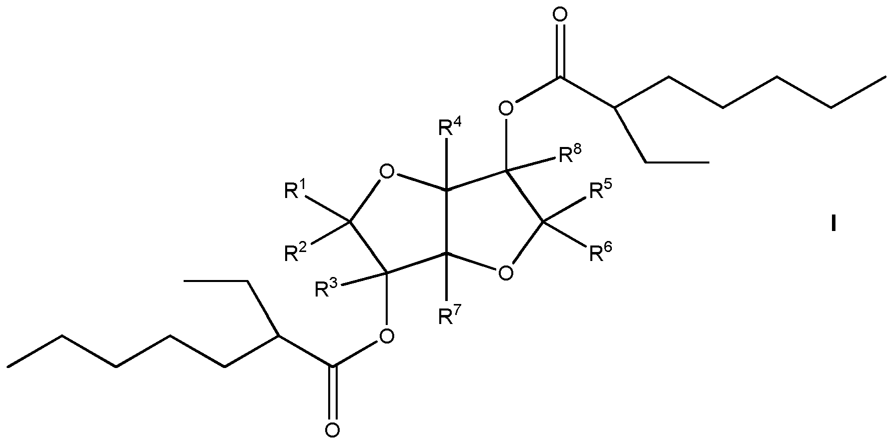 mit R¹ bis R⁸ = H oder Alkylgruppe mit 1 bis 6 Kohlenstoffatomen, wobei die Reste R¹ bis R⁸ gleich oder unterschiedlich sein können.

2. Verbindung nach Anspruch 1,
wobei sich die Reste R¹ und R⁶, sowie R² und R⁵ jeweils entsprechen.

3. Verbindung nach einem der Ansprüche 1 oder 2,
wobei die Reste R¹, R², R⁵, R⁶ jeweils H sind.

4. Verbindung nach einem der Ansprüche 1 bis 3,
wobei sich die Reste R⁴ und R⁷ entsprechen.

5. Verbindung nach einem der Ansprüche 1 bis 4,
wobei die Reste R⁴, R⁷ jeweils H sind.

6. Verbindung nach einem der Ansprüche 1 bis 5,
wobei die Reste R¹ bis R⁸ jeweils H sind.

7. Verbindung nach einem der Ansprüche 1 bis 5,
wobei mindestens einer der Reste R¹ bis R⁸ nicht H ist.

8. Verbindung nach einem der Ansprüche 1 bis 7,
wobei die Verbindung ein Inversionszentrum aufweist.

9. Verbindung nach einem der Ansprüche 1 bis 7,
wobei die Verbindung kein Inversionszentrum aufweist.

10. Gemisch umfassend:
- eine Verbindung der Formel I nach einem der Ansprüche 1 bis 9,
- ein Polymer.

11. Gemisch nach Anspruch 10,
wobei das Massen-Verhältnis von Polymer zur Verbindung der Formel I von 30 zu 1 bis 1 zu 2,5 beträgt.

12. Gemisch nach einem der Ansprüche 10 oder 11,
zusätzlich umfassend:
- einen Weichmacher, welcher nicht der Formel I entspricht.

13. Gemisch nach Anspruch 12,
wobei das Mol-Verhältnis von dem Weichmacher zu der Verbindung der Formel I von 1 zu 10 bis 10 zu 1 beträgt.

14. Gemisch nach einem der Ansprüche 10 bis 13,
wobei das Polymer Polyvinylchlorid ist.

15. Gemisch nach einem der Ansprüche 10 bis 14,
wobei das Gemisch keine unterschiedlichen Diester aufweist, die sich in der Konstitution zumindest eines der vorhandenen Carbonsäurereste C₈H₁₇COO unterscheiden.

16. Verwendung einer Verbindung nach einem der Ansprüche 1 bis 9 als Weichmacher.

## Claims

1. Compound according to the formula I: 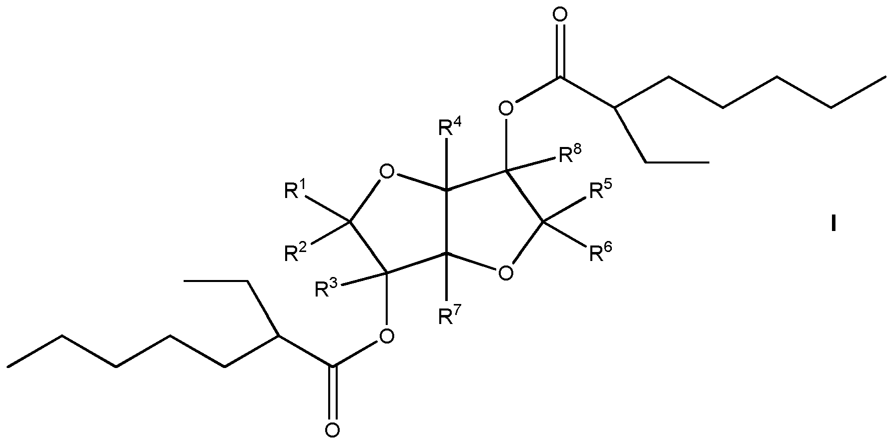 where R¹ to R⁸ = H or alkyl group having from 1 to 6 carbon atoms, where the moieties R¹ to R⁸ can be identical or different.

2. Compound according to Claim 1,
where the moieties R¹ and R⁶, and also R² and R⁵, are respectively identical.

3. Compound according to Claim 1 or 2,
where the moieties R¹, R², R⁵ and R⁶ are respectively H.

4. Compound according to any of Claims 1 to 3, where the moieties R⁴ and R⁷ are identical.

5. Compound according to any of Claims 1 to 4, where the moieties R⁴ and R⁷ are respectively H.

6. Compound according to any of Claims 1 to 5, where the moieties R¹ to R⁸ are respectively H.

7. Compound according to any of Claims 1 to 5, where at least one of the moieties R¹ to R⁸ is not H.

8. Compound according to any of Claims 1 to 7, where the compound has an inversion centre.

9. Compound according to any of Claims 1 to 7, where the compound has no inversion centre.

10. Mixture comprising:
- a compound of the formula I according to any of Claims 1 to 9,
- a polymer.

11. Mixture according to Claim 10,
where the ratio by mass of polymer to the compound of the formula I is from 30:1 to 1:2.5.

12. Mixture according to Claim 10 or 11,
also comprising:
- a plasticizer which does not correspond to the formula I.

13. Mixture according to Claim 12,
where the molar ratio of the plasticizer to the compound of the formula I is from 1:10 to 10:1.

14. Mixture according to any of Claims 10 to 13, where the polymer is polyvinyl chloride.

15. Mixture according to any of Claims 10 to 14, where the mixture does not comprise any different diesters which differ in the constitution of at least one of the carboxylic acid moieties C₈H₁₇COO present.

16. Use of a compound according to any of Claims 1 to 9 as plasticizer.

## Revendications

1. Composé selon la formule I: 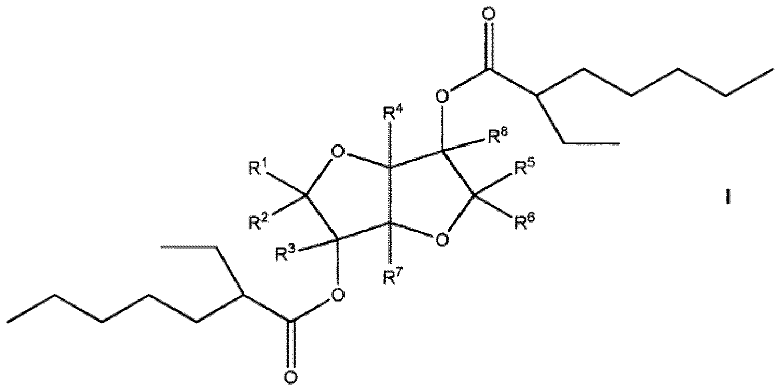 avec R¹ à R⁸ = H ou un groupe alkyle de 1 à 6 atomes de carbone, les radicaux R¹ à R⁸ pouvant être identiques ou différents.

2. Composé selon la revendication 1, dans lequel les radicaux R¹ et R⁶, ainsi que R² et R⁵ se correspondent respectivement.

3. Composé selon l'une quelconque des revendications 1 ou 2, dans lequel les radicaux R¹, R², R⁵, R⁶ représentent chacun H.

4. Composé selon l'une quelconque des revendications 1 à 3, dans lequel les radicaux R⁴ et R⁷ se correspondent.

5. Composé selon l'une quelconque des revendications 1 à 4, dans lequel les radicaux R⁴, R⁷ représentent chacun H.

6. Composé selon l'une quelconque des revendications 1 à 5, dans lequel les radicaux R¹ à R⁸ représentent chacun H.

7. Composé selon l'une quelconque des revendications 1 à 5, dans lequel au moins un des radicaux R¹ à R⁸ ne représente pas H.

8. Composé selon l'une quelconque des revendications 1 à 7, dans lequel le composé comprend un centre d'inversion.

9. Composé selon l'une quelconque des revendications 1 à 7, dans lequel le composé ne comprend pas de centre d'inversion.

10. Mélange, comprenant :
- un composé de formule I selon l'une quelconque des revendications 1 à 9,
- un polymère.

11. Mélange selon la revendication 10, dans lequel le rapport en masse entre le polymère et le composé de formule I est de 30 sur 1 à 1 sur 2,5.

12. Mélange selon l'une quelconque des revendications 10 ou 11, comprenant en outre :
- un plastifiant qui ne correspond pas à la formule I.

13. Mélange selon la revendication 12, dans lequel le rapport en moles entre le plastifiant et le composé de formule I est de 1 sur 10 à 10 sur 1.

14. Mélange selon l'une quelconque des revendications 10 à 13, dans lequel le polymère est le polychlorure de vinyle.

15. Mélange selon l'une quelconque des revendications 10 à 14, dans lequel le mélange ne comprend pas de diesters différents, qui diffèrent au niveau de la constitution d'au moins un des radicaux acide carboxylique C₈H₁₇COO présents.

16. Utilisation d'un composé selon l'une quelconque des revendications 1 à 9 en tant que plastifiant.
